# EUROPEAN PATENT SPECIFICATION

(11) **EP 2 765 989 B1**
(45) Date of publication and mention of the grant of the patent: **13.04.2016**
(21) Application number: 12778188.8
(22) Date of filing: 12.10.2012
(51) Int. Cl.: A61K 9/00, A61K 31/465

(54) **METHODS AND COMPOSITIONS FOR RAPID TRANSBUCCAL DELIVERY OF ACTIVE AGENTS**
VERFAHREN UND ZUSAMMENSETZUNGEN ZUR SCHNELLEN TRANSBUCCALEN VERABREICHUNG VON WIRKSTOFFEN
PROCÉDÉS ET COMPOSITIONS POUR ADMINISTRATION RAPIDE D'AGENTS ACTIFS PAR LA BOUCHE

(30) Priority: 13.10.2011 US 201161546702 P
(43) Date of publication of application: 20.08.2014
(73) Proprietor: Jaleva Pharmaceuticals LLC, San Diego, California 92109 (US)
(72) Inventor: BATTAGLIA, Alex, San Diego, California 92130 (US)
(74) Representative: Lee, Nicholas John
(86) International application number: PCT/US2012/060107
(87) International publication number: WO 2013/056159

(56) References cited:
- WO-A1-2005/065640
- WO-A2-2005/009386
- WO-A2-2007/056491
- Anonymous: "Inflammatory bowel disease, from bench to bedside", 2003, Springer Science +Business Media Inc., USA, XP002692057, page 502, lines 8-13, paragraph 3

## Description

### BACKGROUND

The buccal mucosa is a mucous membrane that lines the inside of the cheek. This mucosa has a rich vasculature that makes it a suitable route for oral delivery of drugs. Drugs absorbed in this manner enter the body via the internal jugular vein, thereby bypassing first-pass liver metabolism and gastric/intestinal enzyme-mediated degradation.

Challenges exist, however, for effective drug delivery through the buccal mucosa. The buccal mucosa contains a 70-micron mucus coating that presents a barrier to drug entry. In addition, the body produces around 750-1,000 cc of saliva daily. This high volume of saliva, which is constantly turned over, causes drugs to be cleared from the oral cavity at a high rate. As a result of this low retention rate, the average bioavailability fraction for transbuccal delivery systems is typically 10% or less. Harris, D. et al. Drug delivery via the mucous membranes of the oral cavity, J. Pharm. Sci., 81:1-10 (1992).

To address this problem, different dosage delivery forms have been developed, including quick-dissolving tablets, waters and films, immediate-release and metered-dose sprays, oral and sublingual rapid-melt and slow-melt tablets, medicated gums and lozenges, mucoadhesive bioerodable preformed discs, controlled release intraoral delivery devices and microparticle technology. Despite these efforts, only a small fraction of drug released from these dosage forms is absorbed at a slow rate by the tissues in the oral cavity. Part of the reason for such a small level and slow rate of absorption is that many of these dosage delivery forms are polymer-based, and cross-linking polymers cause drug trapping and slow drug delivery.

For example, oral pills typically provide a Tmax (i.e., time for a drug to reach maximal plasma concentration in the subject) of over an hour due to gastric/intestinal enzyme-mediated degradation and first-pass liver metabolism. Additionally, the only oral transmucosal nicotine products marketed in the U.S. are nicotine gums, tabs and lozenges. All of these delivery forms have a typical onset time of 30 minutes, due to slow release and swallowing of medication. Henningfield, J.E. et al., Nicotine delivery kinetics and abuse liability, J. Consult. Clin. Psychol., 61(5):743-750(1993). As a results of their slow onset, these delivery forms do not provide an adequate reduction in craving for cigarettes and their long-term efficacy is only 3-8%. Shiftman, S. et al. Real-word efficacy of prescription and over-the-counter nicotine replacement therapy, Addiction, 97(5):505-516 (2002). Moreover, these delivery forms are associated with a variety of side effects including nausea, vomiting, throat and stomach irritation, indigestion and hiccups due to swallowing of the nicotine instead of absorption in the oral cavity. Shiffman, S. et al. Physicians' counseling of patients when prescribing nicotine replacement therapy, Addict. Behav., 32(4):728-39 (2007) further relevant prior art includes WO 2005/00 9386.

### BRIEF DESCRIPTION OF THE FIGURES

Figure 1 shows human pharmacokinetic data comparing serum nicotine plasma concentrations (ng/ml) over time (min.) after (a) buccal administration of a nicotine resin composition as described herein (◆) and (b) administration of Nicorette® nicotine gum (■).
Figure 2 shows the accumulated *in vitro* diffusion of nicotine across human buccal cells after application of a transbuccal dose of a nicotine resin composition as described herein (▲) or a nicotine nasal spray (◆) (peak area, mAU v. time (min.)).
Figure 3 shows the onset of sedation with relief of anxiety (corrected sedation scale score) after (a) transbuccal administration of an alprazolam resin composition as described herein (peak at less than 10 min.) or (b) oral administration of Xanax® alprazolam oral pill (peak at about 50 min.).
Figure 4 shows the accumulated *in vitro* diffusion of zolmitriptan across human buccal cells after application of a transbuccal dose of a zolmitriptan resin composition as described herein (◆) or after administration of Zomig® zolmitriptan nasal spray (A) (peak area, mAU v. time (min.)).
Figure 5 shows the accumulated *in vitro* diffusion of ketorolac across human buccal cells after application of a transbuccal dose of a ketorolac resin composition as described herein (◆) or after administration of Sprix® ketorolac nasal spray (A) (peak area, AUC v. time (min.)).
Figure 6 shows canine pharmacokinetic data (ng/ml vs. minutes) after transbuccal administration of a nicotine resin composition as described herein.
Figure 7 shows canine pharmacokinetic data (ng/ml vs. minutes) after oral administration of a Commit® nicotine lozenge.
Figure 8 shows canine pharmacokinetic data (ng/ml vs. minutes) after transbuccal administration of a non-resin nicotine composition formulated in ethanol.

### SUMMARY OF THE INVENTION

In one aspect, the invention provides a pharmaceutical composition comprising (i) a pharmaceutically active agent selected from the group consisting of nicotine, alprazolam, zolmitriptan, ketorolac, dihydroergotamine, fentanyl, sildenafil, vardenafil, desloratidine and ondasetron, (ii) a resin comprising a benzoin gum, (iii) a volatile solvent, and optionally (iv) water, for use in a method for rapid transbuccal delivery of the active agent into the bloodstream, wherein the composition is for buccal administration to a subject in need thereof.

In some embodiments, the method results in a Tmax of the active agent of less than 60 minutes, less than 45 minutes, less than 30 minutes, less than 20 minutes, less than 10 minutes, less than 5 minutes, or less. In some embodiments, the method results in an onset of therapeutic effect within a time period selected from the group consisting of within 60 minutes or less, within 45 minutes or less, within 30 minutes or less, within 20 minutes or less, within 10 minutes or less, within 5 minutes or less, or sooner. In some embodiments, the method results in therapeutic serum levels of the active agent within a time period selected from the group consisting of within 60 minutes or less, within 45 minutes or less, within 30 minutes or less, within 20 minutes or less, within 10 minutes or less, within 5 minutes or less, or sooner.

In another aspect, the invention provides a pharmaceutical composition for rapid transbuccal delivery of an active agent into the bloodstream, comprising (i) a pharmaceutically active agent selected from the group consisting of nicotine, alprazolam, zolmitriptan, ketorolac, dihydroergotamine, fentanyl, sildenafil, vardenafil, desloratidine and ondasetron, (ii) a resin comprising a benzoin gum, (iii) a volatile solvent, and optionally (iv) water.

Preferred features of the invention are set out in the dependent claims herein.

In any embodiments of the methods and compositions described herein, the resin comprises a benzoin gum. In specific embodiments, the composition may comprise 1-90% resin, such as 1%-25% resin, such as 17%-24% resin. In any embodiments of the methods and compositions described herein, the volatile solvent may comprise ethanol.

In any embodiments of the methods and compositions described herein, the composition may further comprise a penetration enhancer.

In any embodiments of the methods and compositions described herein, the composition may be applied in the form of a paste, a liquid, a semi-solid, a gel, a suspension and an emulsion.

### DETAILED DESCRIPTION

Technical and scientific terms used herein have the meanings commonly understood by one of ordinary skill in the art to which the present invention pertains, unless otherwise defined. Reference is made herein to various methodologies known to those of ordinary skill in the art.

Any suitable materials and/or methods known to those of ordinary skill in the art can be utilized in carrying out the present invention. However, specific materials and methods are described. Materials, reagents and the like to which reference is made in the following description and examples are obtainable from commercial sources, unless otherwise noted.

As used herein, the singular forms "a," "an," and "the" designate both the singular and the plural, unless expressly stated to designate the singular only.

The term "about" and the use of ranges in general, whether or not qualified by the term about, means that the number comprehended is not limited to the exact number set forth herein, and is intended to refer to ranges substantially within the quoted range while not departing from the scope of the invention. As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

The phrase "substantially free" as used herein generally means that the described composition (e.g., pharmaceutical composition, etc.) comprises less than about 5%, less than about 3%, or less than about 1%, by weight, based on the total weight of the composition at issue, of the excluded component.

As used herein "subject" or "patient" denotes any animal in need of therapy, including humans. For example, a subject may be suffering from or at risk of developing a condition that can be treated or prevented with a pharmaceutically active agent, or may be administering a pharmaceutically active agent for health maintenance purposes.

As used herein, the phrases "therapeutically effective amount" and "therapeutic level" mean that drug dosage or plasma concentration in a subject, respectively, that provides the specific pharmacological response for which the active agent is administered in a subject in need of such treatment. It is emphasized that a therapeutically effective amount or therapeutic level of a drug will not always be effective in treating the conditions/diseases described herein, even though such dosage is deemed to be a therapeutically effective amount by those of skill in the art.

Pharmaceutical compositions comprising a resin for use in the oral mucosa, such as for buccal delivery, are stable compositions that can provide a safe, effective and inexpensive method of delivering medications transmucosally. It has surprisingly been found that pharmaceutical compositions comprising (i) a pharmaceutically active agent, (ii) a resin, (iii) a volatile solvent, and optionally (iv) water can achieve rapid transbuccal delivery of an active agent into the bloodstream, such as achieving a Tmax of the active agent of less than 60 minutes, less than 45 minutes, less than 30 minutes, less than 20 minutes, less than 10 minutes, less than 5 minutes, or less. At the same time, these compositions provide a controlled, sustained drug release with good oral bioavailability, such as larger than 10%. In some embodiments, these compositions can eliminate self-regulation when administering a drug and reduce the variance in the amount of drug being delivered. Similarly, in some embodiments, the compositions can reduce the side effects seen with the foregoing delivery forms. In some embodiments, these advantages are provided by allowing the compositions to remain in place on the buccal mucosa and allowing the compositions to release an active agent into the mucosa at a controlled rate over a sustained period of time.

It also has been determined that the methods described herein (e.g., comprising application of the resin compositions described herein to buccal mucosa) can achieve a rapid (early) onset of therapeutic effect. In some embodiments, the onset of therapeutic effect occurs earlier than Tmax. This result can be measured directly, e.g., by assessing clinical responses, or indirectly, i.e., by determining the time it takes to achieve serum levels of the active agent that are associated with therapeutic efficacy (e.g., based on information known in the art from, for example, oral or nasal products). While not wanting to be bound by any theory, it is believed that the earlier onset of therapeutic effect may be associated with the immediate delivery into the bloodstream which is observed, for example, in serum drawn form the internal jugular vein nearly immediately after buccal application of the resin compositions described herein. While not wanting to be bound by any theory, the (early) onset of therapeutic effect may be independent of Tmax because, for example, some of the resin composition may be swallowed, leading to delivery via the gastrointestinal tract, which may contribute to serum levels of active agent at later time points after administration, leading to a later Tmax, despite early achievement of therapeutic serum levels.

Thus, in some embodiments, the methods described herein result in an earlier onset of therapeutic effect than is achieved by oral delivery of the same pharmaceutically active agent. In some embodiments, the methods described herein result in an earlier onset of therapeutic effect than is achieved by nasal delivery of the same pharmaceutically active agent. In some embodiments, the methods described herein result in an onset of therapeutic effect of 45 minutes or less, 30 minutes or less, 20 minutes or less, 10 minutes or less, 5 minutes or less, or sooner, after buccal administration.

In some embodiments, the methods described achieve therapeutic serum levels of the pharmaceutically active agent earlier than is achieved by oral delivery of the same pharmaceutically active agent. In some embodiments, the methods described achieve therapeutic serum levels of the pharmaceutically active agent earlier than is achieved by nasal delivery of the same pharmaceutically active agent. In some embodiments, the methods described herein achieve therapeutic serum levels of the pharmaceutically active agent within 45 minutes or less, 30 minutes or less, 20 minutes or less, 10 minutes or less, 5 minutes or less, or sooner, after buccal administration.

In accordance with some embodiments, a pharmaceutical composition is prepared by combining (1) a pharmaceutically active agent, such as an active agent useful to treat or ameliorate the symptoms of a disease (2) a resin, (3) a volatile solvent, and optionally (4) water. In some embodiments, the resin is dissolved in the volatile solvent. In other embodiments, the composition is prepared as a sticky slurry or solution that can be applied to a site on the mucosal membrane. The consistency of the composition can be varied by adjusting the ratio of solvent to gum in the composition to achieve the desired consistency for application to a particular site. The relative proportions of the resin, the pharmaceutically active agent or agents and the volatile solvent can vary widely, and will depend upon the specific intended use of the composition, including the specific site of application, and the intended duration of application. For example, the desired ratio will depend to some extent on the desired rate of release of the active agent from the composition and the desired stickiness of the composition. As general guidelines, the compositions may have at least about 10%, 20%, 30% or 40% resin, including as much as 50% or 60% or more resin. In specific embodiments, the composition comprises about 1% to 90% resin, about 5% to 90%, about 20% to 85%, about 30% to 70%, or about 40% to 60% resin. In more specific embodiments, the composition comprises about 1% to 25% resin. (These percentages are w/v based on the compositions as formulated, e.g., including solvent.)

In some embodiments, the compositions comprise pharmaceutically active agent, volatile solvent, resin, and, optionally, 10-40% w/v water. In specific embodiments, the resin is benzoin or mastic gum. In specific embodiments, the volatile solvent is selected from the group consisting of alcohols, ketones and ethers, and mixtures thereof, such as ethyl acetate, n-propyl acetate, methanol, ethanol, propanol, isopropanol, isopropyl alcohol, acetone and dimethyl ether, and mixtures thereof, such as isopropyl alcohol. Such compositions may be prepared, for example, by (a) separately preparing (1) a first composition comprising or consisting of a pharmaceutically active agent and optionally further comprising a first volatile solvent and/or water and (2) a second composition comprising a resin, a second volatile solvent and, optionally, water and (b) combining the first and second compositions to obtain a pharmaceutical composition. Optionally, one or more other ingredients, such as dyes, fragrances, flavors, penetration enhancers, and pharmaceutically acceptable carriers, can be added to one or both of the first and second compositions, or can be added after the first and second compositions are combined.

The compositions may include a combination of multiple resins and/or other film-forming agents. In some examples, when a non-resin film-forming agent is used in combination with a resin, the resin is the primary matrix of the composition, i.e., the resin comprises more that 50% of the gum-resin/film-forming agent components of the composition. The compositions may comprise a combination of one or more resins. Each resin may comprise less than 20% of the total composition, although the total amount of all resins combined may exceed 20% of the total composition, and may comprise up to about 90% of the total composition, as noted above. However, the total amount of all resins combined may not exceed 20% of the total composition, and may be as little as 1% to 25% of the total composition, including 17% to 24% of the total composition. Depending on the specific components used, the amounts of each of the resins and/or other film forming agents also may be selected to minimize, reduce or avoid toxicity and/or irritation. (These percentages are w/v based on the compositions as formulated, e.g., including solvent.)

In specific embodiments, the amount of resin in the pharmaceutical composition is in a range of I % to 50% (w/v), including a range of 1% to 35% (w/v), or a range of 1% to 25% (w/v). In specific embodiments, the resin is benzoin and is present in a final concentration in the final composition of from 15% to 50% (w/v), including 15-35% (w/v). In some embodiments the amount of resin is correlated with the desired residence time of the composition on the buccal mucosa, with a higher resin content generally correlated with a longer residence time.

The resin comprises benzoin gum. Exemplary resins include bezoin resinous exudate harvested from Styracaceae trees, including Benzoin Siam from Styrax tonkinesis and Benzoin Sumatra from Styrax benzoin. Tincture of benzoin and benzoin compound tincture is readily available through numerous commercial sources, including many drug stores and suppliers of surgical goods. An optional additional resin that is suitable and commonly used in the medical arts for enhancing the adherence of surgical bandages, is mastic gum, which is harvested from Pistacia lentiscus. A tincture of mastic gum (Mastisol) is produced by Ferndale Laboratories in Ferndale, Mich. and is also available through suppliers of surgical goods. Other optional additional resins that can be used include the resin exudate from Burserceae trees, including Boswellia serrata (also known as Boswellin),

Boswellia dalzielli, Boswellia carteri (gum olibanum) and Canarium luzonicum or Canarium commune (Elemi gum or resin). Additional resinous exudates contemplated from other tree species include Eucalyptus species (Eucalyptus globulus) and Myrtaceae "Tea-tree" species (Melaleuca altemifolia, Leptospermum scoparium, and Kunzea ericoides). Additional resins include Myrrh and Dammar species. Many naturally occurring resins themselves have pharmacological properties, and their topical application may cause irritation in certain patients or exacerbate certain conditions. Prudent choice of resin to be used in preparing a particular pharmacological composition will take into consideration the disorder to be treated and the sensitivities of a particular patient's mucosa. In very specific embodiments, the resin is benzoin.

The compositions are prepared with a volatile solvent. Suitable volatile solvents include lower alcohols such as methanol, ethanol, propanol, and isopropanol, and ketones, such as acetone. Other evaporative compounds may also find use, so long as they are compatible with other components of the pharmacological composition and buccally acceptable to patients. To prepare the composition, the resin(s) can be diluted in the volatile solvent such that the concentration of solvent comprises at least about 40% or 50% (v/v or v/w), more commonly at least about 60%, 70% or 80%, or as much as about 90% of the total composition. In specific embodiments, a tincture of benzoin is used, which is comprised of benzoin in about 60%, 70%, 80% or 90% ethanol.

In some embodiments the volatile solvent(s) may be ethyl acetate, n-propyl acetate, alcohols such as methanol, ethanol, propanol, and isopropanol, isopropyl alcohol, ketones, such as acetone, and ethers such as dimethyl ether. Other evaporative compounds may also find use, so long as they are compatible with other components of the pharmaceutical compositions and topically acceptable to the majority of patients. In specific embodiments where the composition is made by a two-step method as outlined above, a volatile solvent in the first composition, if present, and/or a volatile solvent in the second composition is an alcohol such as ethanol, propanol, and isopropanol. In very specific embodiments, a volatile solvent in the first composition, if present, and/or a volatile solvent in the second composition is isopropanol. In some embodiments, the volatile solvent(s) in the first composition, if present, is(are) the same as the volatile solvent(s) in the second composition. In other embodiments, the volatile solvent(s) in the first composition, if present, is(are) different from the volatile solvent(s) in the second composition. In other embodiments a volatile solvent among a number of volatile solvents in the first composition is the same as a volatile solvent among a number of volatile solvents in the second composition. In other embodiments, a volatile solvent in the first composition, if present, and/or a volatile solvent in the second composition is an isopropyl alcohol mixture in water. In specific embodiments, a volatile solvent in the first composition and/or a volatile solvent in the second composition is a 70% isopropyl alcohol mixture in water.

In specific embodiments where the composition is made by a two-step method as outlined above, the amount of volatile solvent(s) in the first composition, if any, and the amount in the second composition is independently from about 40% to about 98% (v/v), including about 50% to about 95%, or about 60% to about 95%, or about 60% to about 80%, including 50% to 95%, or 60% to 80%, or 60% to 95%, including 40%, 50%, 60%, 70%, 80%, 90% and 98% of the total first or second composition. In specific embodiments where the composition is made by a two-step method as outlined above, the relative amount of the volatile solvent(s) in the first composition, if any, is substantially the same as, or the same as, the relative amount of the volatile solvent(s) in the second composition. In other embodiments, the difference between the relative amount of volatile solvent(s) in the first and second compositions is about 25% or less, about 20% or less, about 15% or less, about 5% or less, or about 1 % or less, all w/v%. In specific embodiments, the difference between the relative amount of volatile solvent(s) in the first and second compositions is 25% or less, 20% or less, 15% or less, 5% or less, or 1% or less, all w/v %. In specific embodiments, the relative amount of the volatile solvent(s) in each of the first composition and the second composition is independently from about 60% to about 80%, such as from 60% to 80%, all w/v %. For example, the first composition may comprise 80% volatile solvent while the second composition may comprise 60% volatile solvent (e.g., a difference of 20%). If the difference between the relative amount of solvent(s) in the first and second compositions is too great, precipitation of some pharmaceutically active agent may occur. In specific embodiments, the volatile solvent in both the first and second compositions is isopropyl alcohol. In other embodiments, the volatile solvent in both the first and second compositions is isopropyl alcohol and combining the first and second compositions results in a final 70% (v/v) concentration of isopropyl alcohol.

In specific embodiments, the amount of volatile solvent in the final composition is in a range of 60% to 99% (v/v).

During use, the volatile solvent evaporates upon application to the buccal mucosa of the subject. In some embodiments, the evaporation results in the formation of a hydrophobic coating on the buccal mucosa, which coating comprises resin and pharmaceutically active agent. In some embodiments, the composition can remain at the site of application for at least 6 hours, including as long as 8, 10 or 12 hours, as long as 16, 18 or 20 hours, and, for certain treatments, as long as 24, 36 or 72 hours, or even longer, prior to removal. The time of treatment desired is based at least in part upon the nature of the condition to be treated and the pharmaceutical agent(s) that are being used.

In specific embodiments, the pharmaceutical composition comprises water is in a range of 1% to 40% (v/v), including water in a range of 1% to 25% (v/v), including a final amount of water of up to about 25% or up to about 40%, such as up to 25% or up to 40%. If too much water is present, the composition may exhibit instability which may be observed, for example, by the formation of precipitates or the loss of intrinsic properties of the resin. The amount of water that can be present without the formation of unacceptable levels of precipitates may vary with the purity of the resin and/or the identity of the pharmaceutically active agent, and typically ranges from about 1% to about 25% (v/v) water.

The compositions can be prepared with any pharmaceutically active agent, such as any pharmaceutically active agent that can be delivered transbuccally. In some examples, the composition may include more than one active agent. The amount of pharmaceutically active agent to be used is generally a therapeutically effective amount, and can be adjusted as appropriate to the condition and subject being treated.

In some embodiments, the pharmaceutically active agent is poorly soluble in a typical resin tincture composition comprising volatile solvent and water. In the context of the methods and compositions described herein, the presence and amount of water in the first composition, if any, and the amount of water in the second composition may be selected and controlled to address this issue. For example, in some embodiments, the presence and amount of water in the first composition, if any, facilitates dissolution of the active agent when mixed with the second composition, which comprises a resin. In other embodiments, the presence and amount of water in the second composition facilitates dissolution of the active agent when mixed with the first composition.

Additionally or alternatively, the presence and amount of water in the final pharmaceutical composition may be selected and controlled to create an environment in which the pH of the final pharmaceutical composition can be adjusted in order to promote a logD of the pharmaceutically active agent that is suitable or advantageous for transmucosal delivery. For example, for transmucosal applications, a lower log D, such as logD of about 1 or less may facilitate or enhance transmucosal delivery, such as by promoting diffusion through the mucosal membranes. Thus, in some embodiments, the presence and amount of water in the final composition may be selected and controlled such that the pH can be adjusted such that the logD of the pharmaceutically active agent is about 1 or less, about 0 or less, about -1 or less, about -2 or less, or less. The pH of the composition can be adjusted by methods known in the art, such as by using a base or acid, such as by adding 1-10 N NaOH or 1-6 N HCl. For example, the pH of the composition may be adjusted to about 7.4 for the following pharmaceutically active agents (logD): nicotine (logD of -0.62); desloratadine (logD of 0.84); naratriptan (logD of -0.85); ondansetron (logD of 1.2). These parameters are exemplary, but non-limiting. For example, a composition suitable for transmucosal delivery of vardenafil can be prepared even though the logD of vardenafil is greater than 1. The logD of vardenafil is 3.71 at pH 7.4, but is 2.26 at pH 5.5. Thus, by adjusting the pH of the composition to 5.5, the transmucosal delivery of vardenafil may be enhanced. Similarly, while the logD of sildenafil is 2.26 at pH of 7.4, sildenafil has a logD of 1.83 at pH 5.5. Thus, by adjusting the pH of the pharmaceutical composition to 5.5, the transmucosal delivery of sildenafil may be enhanced.

In some embodiments, particulate matter forms after water is added to the resin and volatile solvent mixture. For example, precipitates have been observed to form in benzoin resin tinctures at a pH of about 6.1 or greater. In specific embodiments, such particulate matter may be removed by allowing the composition to settle, followed by filtering the composition. In specific embodiments, the composition is allowed to settle for about 1 week, including 1 week, or longer. In other specific embodiments, the composition is filtered using a 0.45 micron filter. It has been discovered that the settled, filtered composition exhibits increased stability, including stability to increased pH, and also exhibits increased stability with regard to the pharmaceutically active agent.

In general, the pharmaceutically active agent(s) may be present at a concentration typically used for that active agent in a topical or buccal formulation. In some embodiments, the pharmaceutically active agent(s) constitutes more than 25% of the first composition, including up to 35% or more. In some embodiments, the pharmaceutically active agent constitutes less than about 25% of the first composition, such as from about 0.1 % (w/v) to about 25% (w/v), or from about 0,1% (w/v) to about 20% (w/v), or from about 0.1 % (w/v) to about 15% (w/v), or from about 0.1 % (w/v) to about 10% (w/v), or from about 0.1% (w/v) to about 5% (w/v), or from about 0.1% (w/v) to about 1% (w/v), or from about 0.5% (w/v) to about 25% (w/v), or from about 0.5% (w/v) to about 20% (w/v), or from about 0.5% (w/v) to about 15% (w/v), or from about 0.5% (w/v) to about 10% (w/v), or from about 0.5% (w/v) to about 5% (w/v), or from about 0.5% (w/v) to about 1% (w/v), or from about 1% (w/v) to about 25% (w/v), or from about 1% (w/v) to about 20% (w/v), or from about 1 % (w/v) to about 15% (w/v), or from about 1% (w/v) to about 10% (w/v), or from about 1% (w/v) to about 5% (w/v), or from about 5% (w/v) to about 25% (w/v), or from about 5% (w/v) to about 20% (w/v), or from about 5% (w/v) to about 15% (w/v), or from about 5% (w/v) to about 10% (w/v), or from about 10% (w/v) to about 25% (w/v), or from about 10% (w/v) to about 20% (w/v), or from about 10% (w/v) to about 15% (w/v), or from about 15% (w/v) to about 25% (w/v), or from about 15% (w/v) to about 20% (w/v), or from about 20% (w/v) to about 25% (w/v), including from 0.1-25% (w/v), 0.1-20% (w/v), 0.1-15% (w/v), 0.1-10% (w/v), 0.1-5% (w/v), 0.1-1% (w/v), 0.5-25% (w/v), 0.5-20% (w/v), 0.5-15% (w/v), 0.5-10% (w/v), 0.5-5% (w/v), 0.5-1% (w/v), 1-25% (w/v), 1-20% (w/v), 1-15% (w/v), 1-10% (w/v), 1-5% (w/v), 5-25% (w/v), 5-20% (w/v), 5-15% (w/v), 5-10% (w/v), 10-25% (w/v), 10-20% (w/v), 10-15% (w/v), 15-25% (w/v), 15-20% (w/v) and 20-25% (w/v), including 0.1% (w/v), 0.5% (w/v), 1% (w/v), 5% (w/v), 10% (w/v), 15% (w/v), 20% (w/v or 25% (w/v). However, because the adherent properties of the compositions may provide extended drug exposure and/or controlled drug delivery, in some embodiments a lower concentration can be used while still achieving the desired pharmaceutical effect. In any event, the amount of pharmaceutically active agent can be determined by the skilled artisan.

In some embodiments, the pharmaceutically active agent is present at a dose approved for clinical use and solubilized in a volume of composition of 200 *µ*l or less. In specific embodiments, the pharmaceutically active agent is solubilized in a volume of composition of 25-200 *µ*l.

In some embodiments, the composition may include a penetration enhancer, i.e., a chemical compound that, when included in a formulation, temporarily increases the permeability of the skin to a drug allowing more of the drug to be absorbed in a shorter period of time. Examples of penetration enhancers that can be used include dimethylsulfoxide, n-decyl methyl sulfoxide, N,N-dimethylacetamide, N,N-methyl-2-pyrrolidone and octylphenylpolyethylene glycols. In some embodiments, menthol and/or peppermint oil may function as enhancers. For example, menthol and/or peppermint oil have been shown to function as enhancers for nicotine, fentanyl, zolmitriptan and dihydroergotamine, when formulated in resin compositions as described herein (e.g., comprising resin and volatile solvent). (Data not shown.)

The composition may also include other pharmaceutically acceptable carriers or excipients as needed that do not adversely affect the effectiveness of the drug, or the resinous delivery vehicle and do not damage the buccal mucosa to which it is applied. Suitable pharmaceutical carriers include sterile water; saline, dextrose; dextrose in water or saline; condensation products of castor oil and ethylene oxide combining about 30 to about 35 moles of ethylene oxide per mole of castor oil; liquid acid; lower alkanols; oils such as corn oil; peanut oil, sesame oil and the like, with emulsifiers such as mono- or di-glyceride of a fatty acid, or a phosphatide, e.g., lecithin, and the like; glycols; polyalkylene glycols; aqueous media in the presence of a suspending agent, for example, sodium carboxymethyl-cellulose; sodium alginate; poly(vinyl pyrrolidone); and the like, alone, or with suitable dispensing agents such as lecithin; polyoxyethylene stearate; and the like. The carrier may also contain adjuvants such as preserving stabilizing, wetting, emulsifying agents and the like.

In some embodiments, the composition is applied directly and specifically on the buccal mucosa. As noted above, the composition may initially be prepared in any form suitable for application, such as a paste, a liquid, a semi-solid, a gel, a suspension, an emulsion or the like. To minimize waste, the composition may be applied by painting or swabbing the composition onto the application site or sites. Alternatively, compositions can be formulated for spray application, such as by a pump spray or aerosol device. In some embodiments, the volatile solvent evaporates upon application of the composition, resulting in the formation of a hydrophobic film or coating on the buccal mucosa, wherein the film or coating comprises resin and pharmaceutically active agent. The film or coating may act as a protective barrier in addition to a drug delivery vehicle. In some embodiments, the drying time is 1 minute. However, the drying time is not limited to a particular time, and can be shorted or extended, for example, to 30 seconds, 1.5 minutes, 2 minutes, 2.5 minutes, 3, 3.5 minutes, 4 minutes, 4.5 minutes, 5 minutes, 5.5 minutes, 6 minutes, 6.5 minutes, 7 minutes, 7.5 minutes, 8 minutes, 8.5 minutes, 9 minutes, 9.5 minutes, 10 minutes, or longer. Thus, the composition can provide a sustained, continuous release of the agent or agents, which may endure as long as the composition remains in place. In some embodiments, the composition is applied once per day, twice per day, or three times daily. In other embodiments, the composition is applied less frequently, such as on alternate days, every two days, every three days, or at longer intervals, such as weekly. The composition can be removed at will, such as by application of an appropriate solvent, such as ethanol, or by scrubbing with soap and water. In some embodiments, the composition is not specifically removed. In specific embodiments, the composition erodes gradually over time, such as by dissolving and being swallowed. This can occur over different time periods, such as, from about 20 to 40 minutes, or over a longer or shorter time.

It also has been discovered that the compositions achieve an unexpected cytoprotective effect. In particular, the compositions reduce the percentage of cell death in the mucosal lining. For example, when human cells are exposed to compositions containing alcohol and active ingredient, but without resin, for a period of 2 hours, about 20%-80% of the cells die. However, when resins, such as benzoin gum and mastic gum, are included in the compositions, about 95%-100% of the cells survive. Specific active agents for which such a cytoprotective effect has been shown include clotrimazole, erythromycin, diclofenac, ibuprofen, nicotine, fentanyl, ondansetron, alprazolam, (Data not shown.)

As noted above, in embodiments where the composition is applied to buccal mucosa, the composition can achieve a rapid (early) Tmax (i.e., time for the active agent to reach maximum plasma concentration) and good overall bioavailability of the active agent. While not wanting to be bound by any theory, it is believed that the composition may achieve immediate and intracellular loading of active agent into the bloodstream, such as by way of the internal jugular vein. Indeed, drug is detected in serum drawn form the internal jugular vein nearly immediately after buccal application of the resin compositions described herein. In some embodiments, the active agent is delivered in a more controlled and sustained fashion with less variability than control samples.

In specific embodiments, the compositions exhibit a two-fold increase in bioavailability as compared to other oral dosage forms. For example, a nicotine composition as described herein exhibits a two-fold increase in bioavailability as compared to a nicotine lozenge dosage form. Other embodiments may exhibit 1.25, 1.33, 1.5, 1.66, 1.75, 2, 2,5, 3, or more times the bioavailability of other another oral dosage form.

In some embodiments, increasing the relative amount of resin in the composition results in a higher Tmax, e.g., accelerates the delivery of active agent into the bloodstream. In contrast, compositions without resin do not exhibit mucoadhesion, quickly release active agent and then release active agent at much lower rates as time passes. With the compositions described herein, the Tmax is generally less than 30 minutes, but may vary depending on the active agent. In some embodiments, the Tmax is less than 25 minutes, less than 20 minutes, less than 15 minutes, less than 10 minutes, or less than 5 minutes.

In specific embodiments, the mean Tmax in humans for a pharmaceutical composition comprising nicotine, benzoin gum and ethanol is 10 minutes. In specific embodiments, the mean Tmax in dogs for a pharmaceutical composition comprising alprazolam, benzoin gum and a volatile solvent is about 10 minutes, such as 9 minutes, 10 minutes, or 11 minutes. In specific embodiments, the mean Tmax in dogs for a pharmaceutical composition comprising ondansetron, benzoin gum and a volatile solvent is about 15 minutes, such as 14 minutes, 15 minutes or 16 minutes.

In specific embodiments, the time to achieve therapeutic plasma levels of active agent from a transbuccal composition as described herein for sildenafil is about 30 minutes, such as 30 minutes (as compared to 30-60 minutes for the oral tablet); for vardenafil is about 10 minutes, such as 10 minutes (compared to 15-50 minutes for the oral tablet); for ketorolac is about 20 minutes, such as 20 minutes (compared to about 30-45 minutes for the nasal spray; for desloratidine is about 30 minutes, such as 30-35 minutes, as compared to 120 minutes for the oral tablet; and for ondansetron is about 20 minutes, such as 18-20 minutes, compared to about 100-125 minutes, for the oral tablet. These examples are illustrative only and do not limit the scope of the invention.

### EXAMPLE 1

A human pharmacokinetic study was undertaken to compare a nicotine resin composition as described herein (1 mg of nicotine in 50 *µ*l of 79% ethanol and 21% benzoin) with Nicorette® nicotine gum (2 mg per gum). Specifically, 50 *µ*l of the nicotine resin composition was applied to the buccal mucosa of patients and allowed to dry for 60 seconds. For patients receiving the Nicorette® gum, the patients were allowed to chew the gum for 30 minutes, as instructed on the gum label. Blood was drawn at different time points to determine the Tmax, Cmax and AUC of each patient population. An assessment of nicotine craving also was conducted.

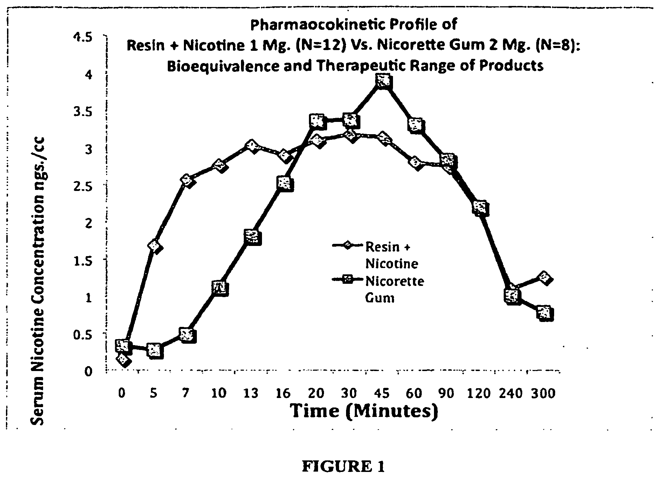

The results of the nicotine resin composition versus Nicorette® gum study are shown in Figure 1, which shows earlier and sustained plasma levels of nicotine in patients treated with the nicotine resin composition as described herein (◆) (n=12) versus the Nicorette® nicotine gum (■) (n =8). Onset of therapeutic effect (e.g., craving reduction) and achievement of therapeutic serum levels (about 2.5 ng/ml) was observed in patients receiving the nicotine resin composition from 7 to 120 minutes after application. In contrast, onset of therapeutic effect was not observed in patients receiving the Nicorette® gum until from 25 to 60 minutes after initiating chewing. These results demonstrate both the pharmacokinetic effect and the direct therapeutic effect of the methods described herein.

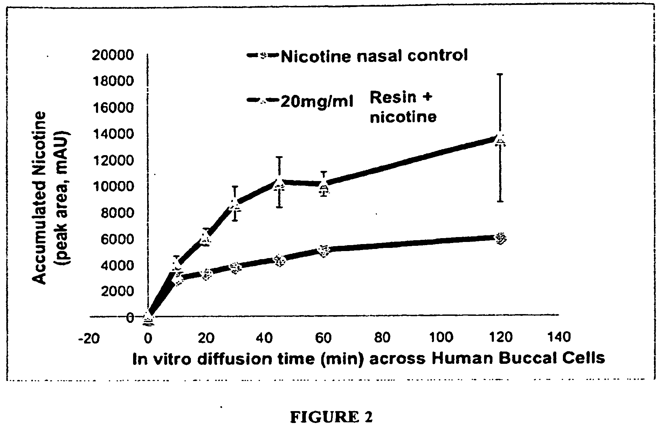

An *in vitro* diffusion study was undertaken to assess the transbuccal delivery of nicotine from a nicotine resin composition as described herein (1 mg of nicotine in 50 *µ*l of 79% ethanol and 21% benzoin) as compared to nicotine nasal spray (1 mg per 100 *µ*l). The results are shown in Figure 2, which shows increased transbuccal delivery from a nicotine resin composition as described herein (▲) versus nicotine nasal spray (◆).

### EXAMPLE 2

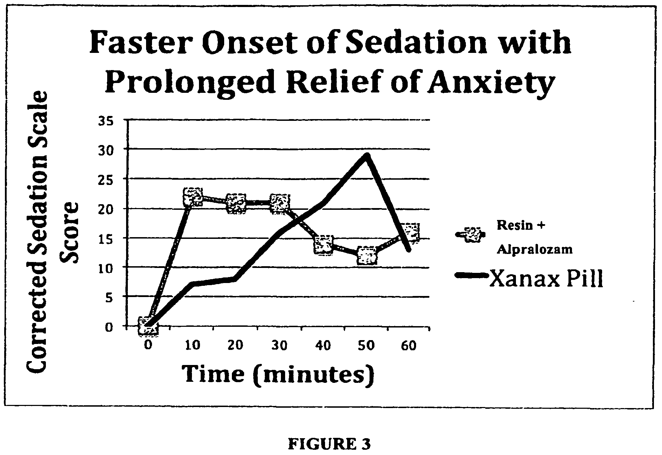

A human efficacy study was undertaken to compare an alprazolam resin composition (0.5 mg/50 *µ*l alprazolam in tincture of benozin, with 0.2 % (w/v) sucralose, 4% (v/v) peppermint oil, 2.1 % (w/v) menthol, at pH 8.1) with Xanax® alprazolam oral pill (0.5 mg dose). Results are shown in Figure 3, which shows an earlier and increased onset of therapeutic effect (sedation with relief of anxiety, corrected sedation scale score) with the alprazolam resin composition as described herein versus Xanax®.

### EXAMPLE 3

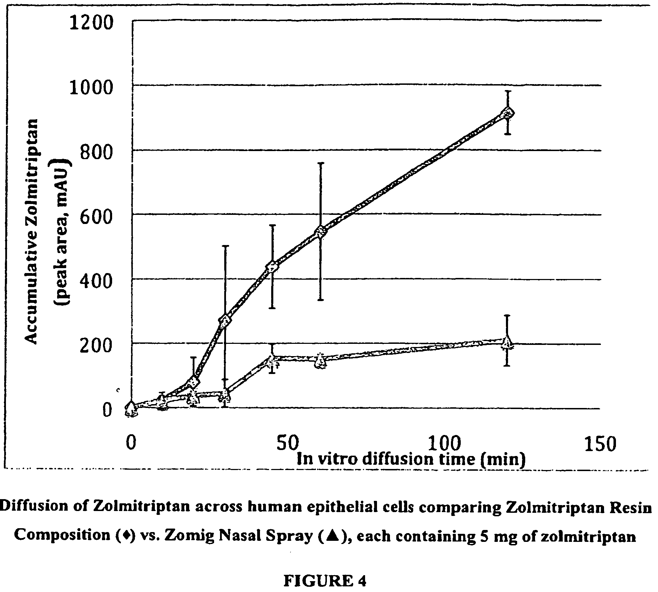

An *in vitro* diffusion study was undertaken to assess the transbuccal delivery of zolmitriptan from a zolmitriptan resin composition (5 mg/50 *µ*l zolmitriptan in tincture of benzoin, with 0.42% (w/v) menthol) as compared to Zomig® zolmitriptan nasal spray (5 mg per 100 *µ*l). The results are shown in Figure 4, which shows increased transbuccal delivery from an administration of 50 *µ*l of the zolmitriptan resin composition as described herein (◆) versus Zomig® (▲).

### EXAMPLE 4

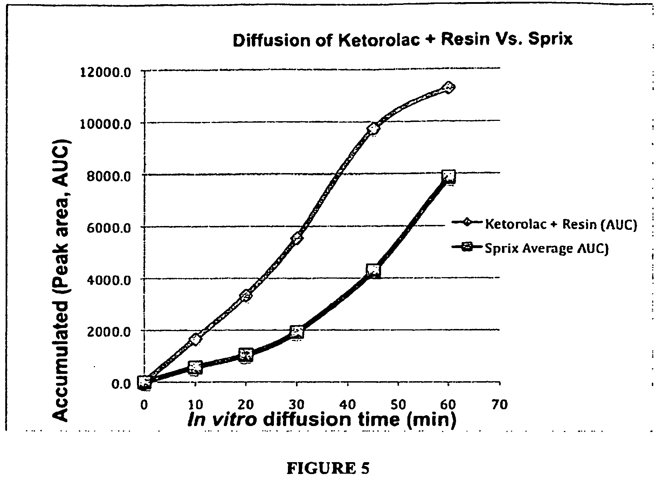

An *in vitro* diffusion study was undertaken to assess the transbuccal delivery of ketorolac from a ketorolac resin composition (15.8 mg/50 *µ*l ketorolac in tincture of benzoin, with 4.2% (w/v) menthol, at pH 6.1) as compared to Sprix® ketorolac nasal spray (15.8 mg per 100 *µ*l). The results are shown in Figure 5, which shows increased transbuccal delivery from a 40 *µ*l administration of ketorolac resin composition as described herein (◆) versus a spray of 80 *µ*l of Sprix®.

Similar *in vitro* diffusion studies were undertaken with dihydroergotamine resin compositions and fentanyl resin compositions, which showed good transbuccal delivery. (Data not shown).

### EXAMPLE 5

A pharmacokinetic study was undertaken in dogs. For each test composition, three beagles were given the body weight equivalent of 1 mg nicotine. One group received a nicotine resin composition as described herein, comprising nicotine in 79% ethanol and 21% benzoin. The composition was placed on the buccal mucosa and allowed to dry for 1 minute. One group received pulverized Commit® lozenge that was placed between the cheek and gums for 1 minute. One group received a formulation comprising nicotine in 79% ethanol ("Vehicle") that was placed on the buccal mucosa and allowed to dry for 1 minute. Blood was drawn from the internal jugular vein at 5, 10, 20, 30, 45, 60, 90 and 120 minutes.

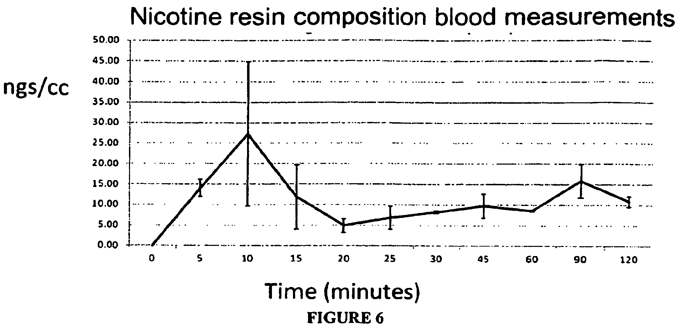

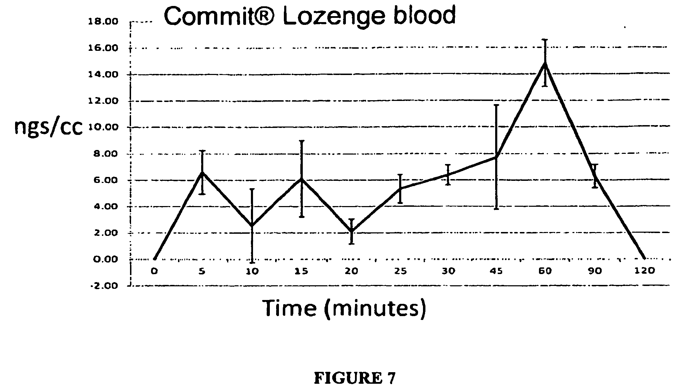

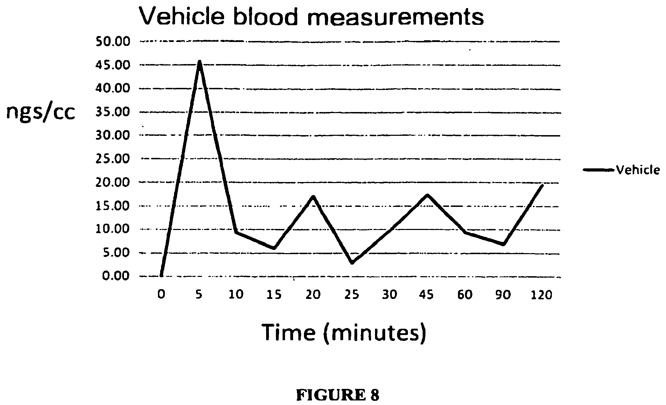

The results are shown in Figures 6-8, respectively, and show that the resin formulation achieves early delivery of nicotine to the bloodstream with a Tₘₐₓ of about 10 minutes, as compared to a Tₘₐₓ of about 60 minutes with the Commit® lozenge. The resin formulation also achieved a sustained delivery of nicotine, over 120 minutes, unlike the Commit® lozenge, which tapered off by 90 minutes. With the non-resin (vehicle) composition, the delivery of nicotine into the bloodstream was both dangerously high and erratic, with a Tₘₐₓ of about 5 minutes and a Cₘₐₓ of about 45.8 ng/ml, compared to the resin compostion (Cₘₐₓ = 27.33 ng/ml) and Commit® lozenge (Cₘₐₓ = 14.8 ng/ml).

### EXAMPLE 6

A pharmacokinetic study was undertaken in dogs to assess and compare transbuccal delivery of several different active agents: sildenafil, vardenafil, ketorolac, desloratidine, ondansetron and alprazolam. Beagle dogs were selected based on their traditional use in pharmacokinetic studies as a non-rodent species, and their buccal mucosa is considered to be equivalent to that of humans.

The active agents were formulated in compositions comprising benzoin resin and volatile solvent, as follows:
Ketorolac: 31.6% (w/v) (15.8 mg ketorolac in 50 *µ*l benzoin, at a pH of 6.1)
Desloratidine: 20% (w/v) (10 mg desloratidine in 50 *µ*l benzoin; 2% peppermint oil and 0.1% sucralose, at a pH of 5.4)
Ondansetron: 8% (w/v) (8 mg ondansetron in 100 *µ*l of 30% (w/v) benzoin; 4.2% L-menthol and 2% peppermint oil, at a pH of 6.1)
Alprazolam: 1% (w/v) (0.5 mg alprazolam in 50 *µ*l benzoin; 0.2% sucralose, 4% peppermint oil and 2.1% L-menthol, at a pH of 8.1)
Sildenafil Mesylate: 25% (w/v) (25 mg sildenafil mesylate in 100 *µ*l of 20% (w/v) benzoin gum; 79% (w/v) ETOH / 21% (w/v) water; 5% L-methanol; 2% peppermint oil and 0.1 % sucralose at a pH of 5.2)
Vardenafil (free base) 13.3% (w/v) (10 mg vardenafil in 75 *µ*l of 20% (w/v) benzoin gum; 79% (w/v) ETOH / 21% (w/v) water and 2% peppermint oil at a pH of 4.1)

These compositions were administered to the buccal mucosa of beagle dogs (n=3) as indicated in Table 1. After each composition was applied, the mouth of the dogs were kept open for 1 minute to allow the composition to dry. Commercial formulations of the same active agents also were administered to beagle dogs (n=3) as indicated in Table 1

**Table 1: Administration**

| **Test Compound** | **Route** | **Dose (mg)/70kg.** |
|---|---|---|
| Sildenafil | Buccal | 50 mg/150uL |
| Vardenafil | Buccal | 20 mg/150uL |
| Ketorolac | Buccal | 31.5 mg/100uL |
| Desloratidine | Buccal | 5 mg/25uL |
| Ondansetron | Buccal | 8 mg/100 uL |
| Alprazolam | Buccal | 0.5 mg/50 uL |
| Viagra® (sildenafil) | PO | 1 pill (50 mg) |
| Levitra® (vardenafil) | PO | 1 pill (10 mg) |
| Clarinex® (desloratidine) | PO | 1 pill (5 mg) |
| Sprix® (ketorolac) | Nasal Spray | 31.5 mg/2 sprays |

Blood was drawn at multiple intervals: pre-dosage; 10, 15, 20, 30, 40, 45, 50, 60, 75, 90, 105, 120, 150, 180, 210, 240 and 360 minutes post-application; and 12, 24 and 48 hours post-application. Approximately 1.0 ml of whole blood was collected at each of the time-points. Blood samples were placed into tubes containing K2 EDTA. The blood was gently mixed to assure distribution of the anti-coagulant. Immediately afterwards, the blood was placed on wet ice or refrigerated, and centrifuged in refrigerated conditions at 4-8° C for approximately 10 minutes at approximately 3,000 RPM. Plasma was then harvested and analyzed at each time-point for levels of the active agent.

Published information on the time of onset of therapeutic effect of the commercial oral or spray product was used to determine the therapeutic serum level. For example, if Viagra® is reported to have a time of onset of therapeutic effect of 30 minutes, the serum level of sildenafil in dogs treated with Viagra® is deemed to be the therapeutic serum level. Then, the time to achieve such serum levels using the transbuccal resin formulation was determined. Results are shown in Table 2.

**Table 2: Results**

| **Test Compound** | **Route** | **Time To Therapeutic Plasma Level** |
|---|---|---|
| Sildenafil | Buccal | 30 min |
| Viagra® (sildenafil) | PO | 30-60 min |
| Vardenafil | Buccal | 10 min |
| Levitra® (vardenafil) | PO | 15-50 min |
| Ketorolac | Buccal | 20 min |
| Sprix® (ketorolac) | Nasal Spray | 30-45 min |
| Desloratidine | Buccal | 33.3 min |
| Clarinex® (desloratidine) | PO | 120 min |
| Ondansetron | Buccal | 18 min |
| Ondansetron | Oral | 103-132 min |
| Alprazolam | Buccal | 9.6-11.3 min |
| Alprazolam | Oral | 30-72 min |

This study showed that with the transbuccal resin compositions, delivery into the bloodstream was nearly immediate, and therapeutic serum levels of drug were achieved much sooner than would have been expected from the reported onset of therapeutic effect of the commercial oral or nasal products.

## Claims

1. A pharmaceutical composition comprising (i) a pharmaceutically active agent selected from the group consisting of nicotine, alprazolam, zolmitriptan, ketorolac, dihydroergotamine, fentanyl, sildenafil, vardenafil, desloratidine and ondasetron, (ii) a resin comprising a benzoin gum, (iii) a volatile solvent, and optionally (iv) water, for use in a method for rapid transbuccal delivery of the active agent into the bloodstream, wherein the composition is for buccal administration to a subject in need thereof.

2. The composition of claim 1, wherein the method results in (a) a Tmax of the active agent selected from the group consisting of less than 60 minutes, less than 45 minutes, less than 30 minutes, less than 20 minutes, less than 10 minutes, or less than 5 minutes, (b) an onset of therapeutic effect within a time period selected from the group consisting of within 60 minutes or less, within 45 minutes or less, within 30 minutes or less, within 20 minutes or less, within 10 minutes or less, within 5 minutes or less, or sooner, or (c) therapeutic serum levels of the active agent within a time period selected from the group consisting of within 60 minutes or less, within 45 minutes or less, within 30 minutes or less, within 20 minutes or less, within 10 minutes or less, within 5 minutes or less, or sooner.

3. A pharmaceutical composition for rapid transbuccal delivery of an active agent into the bloodstream, comprising (i) a pharmaceutically active agent selected from the group consisting of nicotine, alprazolam, zolmitriptan, ketorolac, dihydroergotamine, fentanyl, sildenafil, vardenafil, desloratidine and ondasetron, (ii) a resin comprising a benzoin gum, (iii) a volatile solvent, and optionally (iv) water.

4. The composition of any preceding claim, wherein the composition contains 1%-25% or 17% to 24% resin.

5. The composition of any preceding claim, wherein the composition comprises 1-40% v/v water.

6. The composition of any preceding claim, wherein the volatile solvent comprises ethanol.

7. The composition of any preceding claim, wherein the composition further comprises a penetration enhancer.

8. The composition of claim 7, wherein the penetration enhancer is selected from menthol and peppermint oil.

9. The composition of any preceding claim, wherein the composition is applied in the form of a paste, a liquid, a semi-solid, a gel, a suspension and an emulsion.

10. The composition of any preceding claim, wherein the volatile solvent evaporates, thereby forming a hydrophobic film on the mucosa that comprises resin and active agent.

## Patentansprüche

1. Pharmazeutische Zusammensetzung, umfassend (i) ein pharmazeutisch aktives Mittel, ausgewählt aus der Gruppe, bestehend aus Nikotin, Alprazolam, Zolmitriptan, Ketorolac, Dihydroergotamin, Fentanyl, Sildenafil, Vardenafil, Desloratidin und Ondasetron, (ii) ein Harz, umfassend einen Benzoegummi, (iii) ein flüchtiges Lösungsmittel, und optional (iv) Wasser, zur Verwendung bei einer Methode zur schnellen transbukkalen Beförderung des aktiven Mittels in den Blutstrom, wobei die Zusammensetzung für die bukkale Verabreichung an ein Individuum, das dessen bedarf, vorgesehen ist.

2. Zusammensetzung gemäß Anspruch 1, wobei die Methode zu (a) einer Tₘₐₓ, des aktiven Mittels, ausgewählt aus der Gruppe, bestehend aus weniger als 60 Minuten, weniger als 45 Minuten, weniger als 30 Minuten, weniger als 20 Minuten, weniger als 10 Minuten oder weniger als 5 Minuten, (b) einem Einsetzen der therapeutischen Wirkung innerhalb eines Zeitraums, ausgewählt aus der Gruppe, bestehend aus innerhalb von 60 Minuten oder weniger, innerhalb von 45 Minuten oder weniger, innerhalb von 30 Minuten oder weniger, innerhalb von 20 Minuten oder weniger, innerhalb von 10 Minuten oder weniger, innerhalb von 5 Minuten oder weniger, oder schneller, oder (c) therapeutischen Serumspiegeln des aktiven Mittels innerhalb eines Zeitraums, ausgewählt aus der Gruppe, bestehend aus innerhalb von 60 Minuten oder weniger, innerhalb von 45 Minuten oder weniger, innerhalb von 30 Minuten oder weniger, innerhalb von 20 Minuten oder weniger, innerhalb von 10 Minuten oder weniger, innerhalb von 5 Minuten oder weniger, oder schneller, führt.

3. Pharmazeutische Zusammensetzung zur schnellen transbukkalen Beförderung des aktiven Mittels in den Blutstrom, umfassend (i) ein pharmazeutisch aktives Mittel, ausgewählt aus der Gruppe, bestehend aus Nikotin, Alprazolam, Zolmitriptan, Ketorolac, Dihydroergotamin, Fentanyl, Sildenafil, Vardenafil, Desloratidin und Ondasetron, (ii) ein Harz, umfassend einen Benzoegummi, (iii) ein flüchtiges Lösungsmittel, und optional (iv) Wasser.

4. Zusammensetzung gemäß jedem vorangehenden Anspruch, wobei die Zusammensetzung 1% bis 25% oder 17% bis 24% Harz enthält.

5. Zusammensetzung gemäß jedem vorangehenden Anspruch, wobei die Zusammensetzung 1% bis 40% v/v Wasser enthält.

6. Zusammensetzung gemäß jedem vorangehenden Anspruch, wobei das flüchtige Lösungsmittel Ethanol umfasst.

7. Zusammensetzung gemäß jedem vorangehenden Anspruch, wobei die Zusammensetzung außerdem einen Durchdringungsverstärker umfasst.

8. Zusammensetzung gemäß Anspruch 7, wobei der Durchdringungsverstärker ausgewählt ist aus Menthol und Pfefferminzöl.

9. Zusammensetzung gemäß jedem vorangehenden Anspruch, wobei die Zusammensetzung in der Form einer Paste, einer Flüssigkeit, eines Halbfeststoffs, eines Gels, einer Suspension und einer Emulsion angewendet wird.

10. Zusammensetzung gemäß jedem vorangehenden Anspruch, wobei das flüchtige Lösungsmittel verdampft, wobei es einen hydrophoben Film auf der Schleimhaut bildet, der Harz und aktives Mittel umfasst.

## Revendications

1. Composition pharmaceutique comprenant (i) un agent pharmaceutiquement actif choisi dans le groupe consistant en la nicotine, l'alprazolam, le zolmitriptan, le kétorolac, la dihydroergotamine, le fentanyl, le sildénafil, le vardénafil, la desloratidine et l'ondasétron, (ii) une résine comprenant une gomme benzoïne, (iii) un solvant volatil, et facultativement (iv) de l'eau, pour une utilisation dans une méthode d'administration transbuccale rapide de l'agent actif dans la circulation sanguine, la composition étant destinée à une administration buccale à un sujet en ayant besoin.

2. Composition selon la revendication 1, dans laquelle la méthode conduit à (a) un Tmax de l'agent actif choisi dans le groupe consistant en moins de 60 minutes, moins de 45 minutes, moins de 30 minutes, moins de 20 minutes, moins de 10 minutes ou moins de 5 minutes, (b) un début d'effet thérapeutique dans une période de temps choisie dans le groupe consistant en dans 60 minutes ou moins, dans 45 minutes ou moins, dans 30 minutes ou moins, dans 20 minutes ou moins, dans 10 minutes ou moins, dans 5 minutes ou moins, ou plus tôt, ou (c) des taux sériques thérapeutiques de l'agent actif dans une période de temps choisie dans le groupe consistant en dans 60 minutes ou moins, dans 45 minutes ou moins, dans 30 minutes ou moins, dans 20 minutes ou moins, dans 10 minutes ou moins, dans 5 minutes ou moins, ou plus tôt.

3. Composition pharmaceutique pour une administration transbuccale rapide d'un agent actif dans la circulation sanguine, comprenant (i) un agent pharmaceutiquement actif choisi dans le groupe consistant en la nicotine, l'alprazolam, le zolmitriptan, le kétorolac, la dihydroergotamine, le fentanyl, le sildénafil, le vardénafil, la desloratidine et l'ondasétron, (ii) une résine comprenant une gomme de benzoïne, (iii) un solvant volatil et facultativement (iv) de l'eau.

4. Composition selon l'une quelconque des revendications précédentes, dans laquelle la composition contient 1 % - 25 % ou 17 % à 24 % de résine.

5. Composition selon l'une quelconque des revendications précédentes, dans laquelle la composition comprend 1 - 40 % v/v d'eau.

6. Composition selon l'une quelconque des revendications précédentes, dans laquelle le solvant volatil comprend de l'éthanol.

7. Composition selon l'une quelconque des revendications précédentes, dans laquelle la composition comprend en outre un agent améliorant la pénétration.

8. Composition selon la revendication 7, dans laquelle l'agent améliorant la pénétration est choisi parmi le menthol et l'essence de menthe poivrée.

9. Composition selon l'une quelconque des revendications précédentes, dans laquelle la composition est appliquée sous la forme d'une pâte, d'un liquide, d'un semi-solide, d'un gel, d'une suspension et d'une émulsion.

10. Composition selon l'une quelconque des revendications précédentes, dans laquelle le solvant volatil s'évapore, formant par là un film hydrophobe sur la muqueuse qui comprend la résine et l'agent actif.
